# EUROPÄISCHE PATENTANMELDUNG

(11) **EP 4 464 727 A1**
(43) Veröffentlichungstag der Anmeldung: **20.11.2024**
(21) Anmeldenummer: 23173527.5
(22) Anmeldetag: 16.05.2023
(51) Int. Cl.: C08G 18/02, C07C 67/00

(54) **VERESTERTE ETHANOLAMINE ZUR HERSTELLUNG VON ISOCYANURATPOLYMEREN**

(71) Anmelder: Covestro Deutschland AG, 51373 Leverkusen (DE)
(72) Erfinder: Die Erfindernennung liegt noch nicht vor
(74) Vertreter: Levpat

(57) **Zusammenfassung**

Die vorliegende Erfindung betrifft die Verwendung von veresterten Ethanolaminen als Katalysatoren für die Vernetzung aliphatisch und/oder cycloaliphatisch gebundener Isocyanatgruppen untereinander. Die erfindungsgemäßen Katalysatoren haben den besonderen Vorteil, dass sie thermolatent sind.

## Beschreibung

Die vorliegende Erfindung betrifft die Verwendung von veresterten Ethanolaminen als Katalysatoren für die Vernetzung aliphatisch und/oder cycloaliphatisch gebundener Isocyanatgruppen untereinander. Die erfindungsgemäßen Katalysatoren haben den besonderen Vorteil, dass sie thermolatent sind.

Die Herstellung von Isocyanuratkunststoffen durch die Vernetzung aliphatisch oder cycloaliphatisch gebundener Isocyanatgruppen untereinander, d.h. ohne Beteiligung von Thiol-, Hydroxyl- oder Aminogruppen ist grundsätzlich bekannt. Es ist auch bereits beschrieben, dass derartige Materialien als Polymermatrix für Verbundwerkstoffe einsetzbar sind.

WO 2019/197638 beschreibt Isocyanataddukte von hydroxyfunktionellen tertiären Aminen und die Verwendung dieser Verbindungen als Trimerisierungskatalysatoren. Die Zugabe dieser Addukte zu Polyisocyanaten bewirkt allerdings eine unmittelbare Viskositätserhöhung der damit aktivierten Reaktionsgemische. Diese setzt sich über die angestrebte Nutzungsdauer der Zusammensetzung fort. Viele Herstellungsverfahren für Verbundwerkstoffe mit Polyisocyanuratmatrix, so z.B. das Strangziehverfahren, beruhen darauf, dass ein faserförmiger Füllstoff in einem Tauchbad mit dem Matrixmaterial benetzt wird. Deswegen beschränkt der Viskositätsanstieg die Nutzungsdauer des Reaktionsgemisches, das zum Aufbau der Matrix eingesetzt wird. Dies ist gerade vor dem Hintergrund der Verwendung des Systems im Strangziehverfahren für Verbundwerkstoffe hinderlich, da damit eine optimale Faseranbindung nicht sicher erreicht werden kann.

WO 2019/197639 offenbart die Verwendung der hydroxyfunktionellen tertiären Amine für die Vernetzung von aliphatischen Polyisocyanaten im Strangziehverfahren zur Herstellung von Verbundwerkstoffen. Es werden aber keine Verbindungen offenbart, bei denen die OH-Gruppe dieser Verbindungen verestert ist. Auch bei diesen Verbindungen ist ein störender Viskositätsanstieg des Reaktionsgemisches zu beobachten.

Der vorliegenden Erfindung lag die Aufgabe zugrunde, Trimerisierungskatalysatoren bereitzustellen, die zu einem möglichst geringen Viskositätsanstieg einer Polyisocyanatzusammensetzung führen, eine gute Anbindung der Polyisocyanuratmatrix eines Verbundwerkstoffs an den Füllstoff ermöglichen und zu Polyisocyanuratmatrizes mit verlässlich hohen Glasübergangstemperaturen führen.

Diese Aufgabe wird durch die Ausführungsformen der vorliegenden Erfindung gelöst, die in den Patentansprüchen und in dieser Beschreibung offenbart werden.

In einer ersten Ausführungsform betrifft die vorliegende Erfindung eine Verbindung gemäß Formel (I) 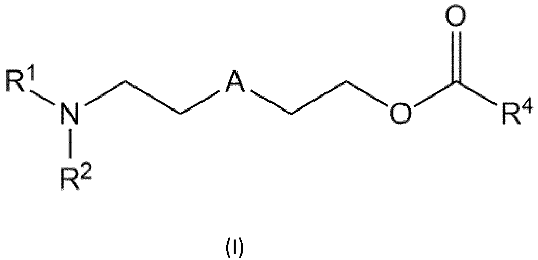
Wobei R¹ und R² unabhängig voneinander ausgewählt sind aus der Gruppe bestehend aus Wasserstoff, Methyl, Ethyl, Propyl, Isopropyl, Butyl und Isobutyl, verzweigtem C5-Alkyl, unverzweigtem C5-Alkyl, verzweigtem C6-Alkyl, unverzweigtem C6-Alkyl, verzweigtem C7-Alkyl und unverzweigtem C7-Alkyl;
A ausgewählt ist aus der Gruppe bestehend aus O, S und NR³, wobei R³ ausgewählt ist aus der Gruppe bestehend aus H, Methyl, Ethyl, Propyl, Isopropyl, Butyl und Isobutyl; und
R⁴ ein verzweigter oder unverzweigter Alkylrest bis zu 7 Kohlenstoffatomen oder Wasserstoff ist.

### Bevorzugte Varianten der Verbindung gemäß Formel (I)

In einer bevorzugten Ausführungsform der vorliegenden Erfindung ist A NR³, wobei R³ ausgewählt ist aus der Gruppe bestehend aus Wasserstoff, Methyl, Ethyl, Propyl, Isopropyl, Butyl und Isobutyl. Bevorzugt ist R³ Methyl oder Ethyl. Besonders bevorzugt ist R³ Methyl.

In einer weiteren bevorzugten Ausführungsform dieser Erfindung ist A Sauerstoff.

In noch einer weiteren bevorzugten Ausführungsform dieser Erfindung ist A Schwefel.

In einer bevorzugten Ausführungsform der vorliegenden Erfindung ist R⁴ ausgewählt aus der Gruppe bestehend aus Wasserstoff, Methyl und Ethyl. Besonders bevorzugt ist R⁴ Wasserstoff oder Methyl.

### Polyisocyanat

Unter einem "Polyisocyanat" wird in der vorliegenden Anmeldung jede Verbindung verstanden, die im Durchschnitt wenigstens 1,8, bevorzugt wenigstens 2,0 und besonders bevorzugt 2,1 Isocyanatgruppen aufweist. Hingegen wird unter einem "Monoisocyanat" eine Verbindung mit durchschnittlich höchstens 1,6 Isocyanatgruppen pro Molekül, insbesondere nur mit einer Isocyanatgruppe pro Molekül verstanden.

Der Begriff "Polyisocyanate" bezeichnet in dieser Anmeldung monomere und/oder oligomere Polyisocyanate gleichermaßen. Zum Verständnis vieler Aspekte der Erfindung ist es jedoch wichtig, zwischen monomeren Diisocyanaten und oligomeren Polyisocyanaten zu unterscheiden. Wenn in dieser Anmeldung von "oligomeren Polyisocyanaten" die Rede ist, dann sind damit Polyisocyanate gemeint, die aus mindestens zwei monomeren Diisocyanatmolekülen aufgebaut sind, d.h. es sind Verbindungen, die ein Reaktionsprodukt aus mindestens zwei monomeren Diisocyanatmolekülen darstellen oder enthalten.

### Oligomere Isocyanate

Oligomere Isocyanate werden durch "Modifizierung" eines monomeren Isocyanats erhalten. "Modifizierung" bedeutet dabei die Reaktion monomerer Isocyanate zu oligomeren Isocyanaten mit Uretdion-, Isocyanurat-, Allophanat-, Biuret-, Iminooxadiazindion- und/oder Oxadiazintrionstruktur. Als Edukte für die Herstellung oligomerer Isocyanate werden bevorzugt Diisocyanate verwendet.

So ist z.B. Hexamethylendiisocyanat (HDI) ein "monomeres Diisocyanat", da es zwei Isocyanat-Gruppen enthält und kein Reaktionsprodukt aus mindestens zwei Polyisocyanatmolekülen darstellt:

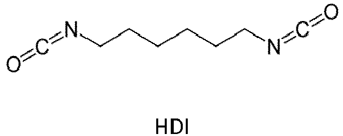

Reaktionsprodukte aus mindestens zwei HDI-Molekülen, die immer noch über mindestens zwei Isocyanat-Gruppen verfügen, sind demgegenüber "oligomere Polyisocyanate" im Sinne der Erfindung. Vertreter solcher "oligomerer Polyisocyanate" sind ausgehend von dem monomeren HDI z.B. das HDI-Isocyanurat und das HDI-Biuret, die jeweils aus drei monomeren HDI Bausteinen aufgebaut sind:

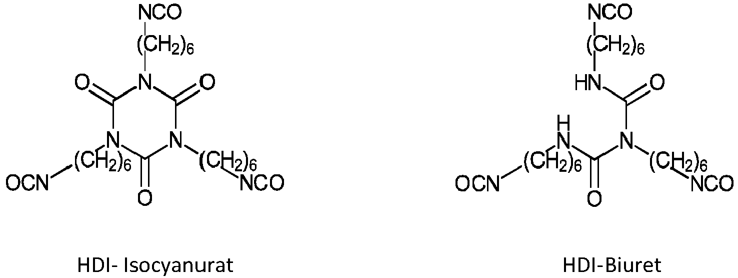

### (idealisierte Strukturformeln)

Herstellverfahren für oligomere Polyisocyanate mit Uretdion-, Isocyanurat-, Allophanat-, Biuret-, Iminooxadiazindion- und/oder Oxadiazintrionstruktur sind beispielsweise in J. Prakt. Chem. 336 (1994) 185 - 200, in DE-A 1 670 666, DE-A 1 954 093, DE-A 2 414 413, DE-A 2 452 532, DE-A 2 641 380, DE-A 3 700 209, DE-A 3 900 053 und DE-A 3 928 503 oder in EP-A 0 336 205, EP A 0 339 396 und EP-A 0 798 299 beschrieben.

Besonders bevorzugt werden die weiter unten in dieser Anmeldung definierten monomeren Isocyanate als Ausgangsstoffe der Modifizierung verwendet.

Die erfindungsgemäße polymerisierbare Zusammensetzung kann oligomere und polymere Polyisocyanate in beliebigen Mischungsverhältnissen enthalten. Grundsätzlich sind aus Gründen der Arbeitssicherheit polymerisierbare Zusammensetzungen bevorzugt, deren Polyisocyanatkomponente, d.h. die Gesamtheit aller in besagter Zusammensetzung enthaltenen Polyisocyanate, zu wenigstens 90 Gew.-%, bevorzugt wenigstens 95 Gew.-% und stärker bevorzugt wenigstens 98 Gew.-% aus oligomeren Polyisocyanaten bestehen. Soweit gewünscht, beispielsweise zur Absenkung der Viskosität der polymerisierbaren Zusammensetzung, kann die Polyisocyanatkomponente aber auch bis 20 Gew.-% oder bevorzugt bis zu 50 Gew.-% monomere Polyisocyanate enthalten.

### Isocyanate mit aliphatisch gebunden Isocyanatgruppen

Bei einem Isocyanat mit aliphatisch gebundenen Isocyanatgruppen sind alle Isocyanatgruppen an ein Kohlenstoffatom gebunden, das Teil einer offenen Kohlenstoffkette ist. Diese kann an einer oder mehreren Stellen ungesättigt sein. Die aliphatisch gebundene Isocyanatgruppe oder - im Fall von Polyisocyanaten - die aliphatisch gebundenen Isocyanatgruppen sind vorzugsweise an den terminalen Kohlenstoffatomen der Kohlenstoffkette gebunden.

Erfindungsgemäß besonders geeignete Polyisocyanate mit aliphatisch gebundenen Isocyanatgruppen sind 1,4-Diisocyanatobutan (BDI), 1,5-Diisocyanatopentan (PDI), 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan und 1,10-Diisocyanatodecan.

### Isocyanate mit cycloaliphatisch gebunden Isocyanatgruppen

Bei einem Isocyanat mit cycloaliphatisch gebundenen Isocyanatgruppen sind alle Isocyanatgruppen an Kohlenstoffatome gebunden, die Teil eines geschlossenen Rings aus Kohlenstoffatomen sind. Dieser Ring kann an einer oder mehreren Stellen ungesättigt sein, solange er durch das Vorliegen von Doppelbindungen keinen aromatischen Charakter erhält.

Erfindungsgemäß besonders geeignete Polyisocyanate mit cycloaliphatisch gebundenen Isocyanatgruppen sind 1,3- und 1,4-Diisocyanatocyclohexan, 1,4-Diisocyanato-3,3,5-trimethylcyclohexan, 1,3-Diisocyanato-2-methylcyclohexan, 1,3-Diisocyanato-4-methylcyclohexan, 1-Isocyanato-3,3,5-trimethyl-5-isocyanatomethyl-cyclohexan Isophorondiisocyanat; (IPDI), 1-Isocyanato-1-methyl-4(3)-isocyanatomethylcyclohexan, 2,4'- und 4,4'-Diisocyanatodicyclohexylmethan(H12MDI), 1,3-und 1,4-Bis(isocyanatomethyl)cyclohexan, Bis-(isocyanatomethyl)-norbornan (NBDI), 4,4'-Diisocyanato-3,3'-dimethyldicyclohexylmethan, 4,4'-Diisocyanato-3,3',5,5'-tetramethyl-dicyclohexylmethan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetra-methyl-1,1'-bi(cyclohexyl), 1,8-Diisocyanato-p-menthan, 1,3-Diisocyanato-adamantan und 1,3-Dimethyl-5,7-diisocyanatoadamantan .

### Isocyanate mit araliphatisch gebunden Isocyanatgruppen

Bei einem Isocyanat mit araliphatisch gebundenen Isocyanatgruppen sind alle Isocyanatgruppen an Methylenreste gebunden, der ihrerseits an einen aromatischen Ring gebunden sind.

Erfindungsgemäß besonders geeignete Polyisocyanate mit araliphatisch gebundenen Isocyanatgruppen sind 1,3- und 1,4-Bis-(iso-cyanatomethyl)benzol (Xyxlylendiisocyanat; XDI), 1,3- und 1,4-Bis(1-isocyanato-1-methyl,ethyl)-benzol (TMXDI) und Bis(4-(1-isocyanato-1-methylethyl)phenyl)-carbonat.

Erfindungsgemäß kann die polymerisierbare Zusammensetzung beliebige Mischungen der oben genannten Isocyanate in monomerer und/oder oligomerer Form enthalten.

### Isocyanat mit aromatisch gebundener Isocyanatgruppe

Bei einem Isocyanat mit aromatisch gebundener Isocyanatgruppe sind alle Isocyanatgruppen direkt an Kohlenstoffatome gebunden, die Teil eines aromatischen Ringes sind.

Erfindungsgemäß besonders geeignete Isocyanate mit aromatisch gebundenen Isocyanatgruppen sind 2,4- und 2,6-Diisocyanattoluol (TDI), 2,4'- und 4,4'-Diisocyanatodiphenylmethan (MDI) und 1,5-Diisocyanatonaphthalin.

### Monoisocyanate

Erfindungsgemäß besonders geeignete Monoisocyanate sind vorzugsweise ausgewählt aus der Gruppe bestehend aus n-Butylisocyanat, n-Amylisocyanat, n-Hexylisocyanat, n-Heptylisocyanat, n-Octylisocyanat, Undecylisocyanat, Dodecylisocyanat, Tetradecylisocyanat, Cetylisocyanat, Stearylisocyanat, Cyclopentylisocyanat, Cyclohexylisocyanat, 3- bzw. 4-Methylcyclohexylisocyanat, Methylbenzylisocyanat, Methylisocyanat, (Trimethylsilyl)isocyanat, 1-Naphtylisocyanat, 3-Methyl-2-butylisocyanat, 1-(4-Methoxyphenyl)ethylisocyanat, 1-(3-methoxyphenyl)ethylisocyanat, 1-Phenylpropylisocyanat, 2-Octylisocyanat, 2-Heptylisocyanat, 4-butyl-2-methylphenylisocyanat, 3-(Triethoxysilyl)propylisocyanat, 2-Benzyloxycyclohexylisocyanat, 1-(4-Chlorophenyl)ethylisocyanat, 2-Nonylisocyanat, 1-(4-Bromophenyl)ethylisocyanat, 2,1,3-benzothiadiazol-4-ylisocyanat, p-Phenylazophenylisocyanat, Phenylisocyanat, Ethylisocyanat, Chlorsulfonylisocyanat, Allylisocyanat, Benzylisocyanat, Propylisocyanat, Isoproylisocyanat, Furfurylisocyanat, Propylisocyanat, Octadecylisocyanat, Trichloracetylisocyanat, Benzoylisocyanat, Phenethylisocyanat, p-Tolylisocyanat, o-Tolylisocyanat, m-Tolylisocyanat, 3,4-Dimethoxyphenylisocyanat, 2,4-Dimethoxyphenylisocyanat, 3,5-Dimethoxyphenylisocyanat, 2,5-Dimethoxyphenylisocyanat, tert-Butylisocyanat, 3,5-Dimethylphenylisocyanat, 2,6-Dimethylphenylisocyanat, 4-Ethylphenylisocyanat, 4-Methylbenzylisocyanat, 2-Methylbenzylisocyanat, 3-Methylbenzylisocyanat, 4-Methoxyphenylisocyanat, 4-tert-Butylphenylisocyanat, 2-Methoxyphenylisocyanat, 3,4,5-Trimethoxyphenylisocyanat, 2,4-Dimethoxybenzylisocyanat, 4-Phenylbutylisocyanat, 4-Ethylphenethylisocyanat, 4-Methoxybenzylisocyanat, Benzenesulfonylisocyanat, 2-Methoxybenzylisocyanat, 3-Ethoxyphenylisocyanat, 3-Methoxybenzylisocyanat, 2,2-Diphenylethylisocyanat, 1,1,3,3-Tetramethylbutylisocyanat, 2-Ethylhexylisocyanat, 4-Biphenylylisocyanat, 3-Phenylpropylisocyanat, 2,3-Dimethoxyphenethylisocyanat, Decylisocyanat, Cyclohexanmethylisocyanat, 3,4-Methylendioxyphenethylisocyanat, 3,4-Dimethoxyphenethylisocyanat, 5-Indanylisocyanat, Cycloheptylisocyanat, 2-Phenylcyclopropylisocyanat, 1-Cyclohexylethylisocyanat, 4-Nitrophenylisocyanat, 1-Adamantylisocyanat, 2-Nitrophenylisocyanat, 3-Nitrophenylisocyanat, Pyridine-3-isocyanat, Chloracetylisocyanat, 2,6-Diisopropylphenylisocyanat, Hexadecylisocyanat, 4-Acetylphenylisocyanat, 4-Phenoxyphenylisocyanat, 4-Pentylphenylisocyanat, 3-Phenoxyphenylisocyanat, p-Toluenesulfonylisocyanat, 2-Chlorethylisocyanat, 2-Bromphenylisocyanat, 3-Chlorphenylisocyanat, 2-Chlorphenylisocyanat, 4-Bromphenylisocyanat, 4-Chlorphenylisocyanat, 2-Naphthylisocyanat, 4-Fluorophenylisocyanat, 2-Bromethylisocyanat, 4-Cyanophenylisocyanat, 3,4-Dichlorphenylisocyanat, 2,3,4-Trifluorphenylisocyanat, 3-Cyanophenylisocyanat, 2,6-Dichlorphenylisocyanat, Diethoxyphosphinylisocyanat, 2,4-Dichlorphenylisocyanat, 3,3,4,4,5,5,6,6,7,7,8,8,9,9,10,10,10-Heptadecafluordecylisocyanat, 4-Fluorbenzylisocyanat, 2-Fluorphenylisocyanat, 3-Chlorpropylisocyanat, 3-Fluorphenylisocyanat, 4-lodphenylisocyanat, 3,5-Dichlorphenylisocyanat, 4-Chlorbenzenesulfonylisocyanat, 2,4,6-Tribromphenylisocyanat, 2-lodphenylisocyanat, 3,4-Difluorphenylisocyanat, 3-Bromphenylisocyanat, 2,4-Dichlorbenzylisocyanat, 2,5-Difluorphenylisocyanat, 2-Benzylphenylisocyanat, 2-Fluorbenzylisocyanat, 4-Fluorphenethylisocyanat, Pentafluorphenylisocyanat, 2,4-Dichlorphenethylisocyanat, 4-Chlorbenzylisocyanat, Diphenylmethylisocyanat, Tributylzinnisocyanat, 2-Chlorbenzolsulfonylisocyanat, 2-Chlorbenzylisocyanat, 3,3-Diphenylpropylisocyanat, , 3,4,5-Trimethoxybenzylisocyanat, 3-Chlorphenethylisocyanat, 3-Fluorbenzylisocyanat, 2,6-Difluorphenylisocyanat, 3-lodphenylisocyanat, 2,4-Difluorphenylisocyanat, 2-Cyanophenylisocyanat, 2-Fluorphenethylisocyanat, 2-Thienylisocyanat, 3,4-Dichlorbenzylisocyanat, 3,4-Dichlorphenethylisocyanat, 4-Benzylphenylisocyanat, 4-Brombenzylisocyanat, 4-Fluorbenzopsulfonylisocyanat, mPEG5K-lsocyanat, 3,5-Dimethylisoxazol-4-ylisocyanat, 2-Methoxy-5-methylphenylisocyanat, 2-(4-Biphenyl)ethylisocyanat, 2-Ethyl-6-methylphenylisocyanat, 2-Methyl-5-phenyl-3-furylisocyanat, 1-(1-Naphthyl)ethylisocyanat, 3,4-(Methylenedioxy)phenylisocyanat, 2,3-Dihydro-1-benzofuran-5-ylisocyanat, 4-Methoxy-2-nitrophenylisocyanat, 3,5-Bis(trifluormethyl)phenylisocyanat, 4-(Maleinimido)phenylisocyanat, 4-(Dimethylamino)phenylisocyanat, 3-(Trifluormethyl)phenylisocyanat, 4-(Chlorsulfonyl)phenylisocyanat, 3-Isopropenyl-α,α-dimethylbenzylisocyanat, 3-Chlor-4-methylphenylisocyanat, 4-(Trifluormethyl)phenylisocyanat, 2-(Trifluormethyl)phenylisocyanat, 4,4'-Oxybis(phenylisocyanat), 4-(Chlormethyl)phenylisocyanat, 4-Chlor-3-(trifluormethyl)phenylisocyanat, 9H-Fluoren-2-ylisocyanat, 2-(Chlormethyl)phenylisocyanat, 2-Fluor-5-(trifluormethyl)phenylisocyanat, 2-Fluor-3-(trifluormethyl)phenylisocyanat, 4-(Benzyloxy)phenylisocyanat, 4-Fluor-3-(trifluormethyl)phenylisocyanat, 4-Fluor-3-methylphenylisocyanat, 3-Fluor-5-(trifluormethyl)phenylisocyanat, 4-Chlor-2-fluorphenylisocyanat, 5-Fluor-2-methylphenylisocyanat, 2,3-Dimethyl-6-nitrophenylisocyanat, 2-(Trifluormethoxy)phenylisocyanat, 2-Fluor-5-methylphenylisocyanat, 4-(Difluormethoxy)phenylisocyanat, 4-Methyl-2-nitrophenylisocyanat, 3-Fluor-2-methylphenylisocyanat, 4-(Trifluormethylthio)phenylisocyanat, 4-Fluor-2-(trifluormethyl)phenylisocyanat, 1-(4-Fluorphenyl)ethylisocyanat, 1-Benzothiophen-5-ylisocyanat, 2-(Difluormethoxy)phenylisocyanat, 2-(Thien-2-yl)ethylisocyanat, 2-Brom-4,6-difluorphenylisocyanat, 2-Chlor-4,6-dimethylphenylisocyanat, 2-Chlor-4-(trifluoromethyl)phenylisocyanat, 2-Chlor-4-(trifluormethylthio)phenylisocyanat, 2-Chlor-5-methylphenylisocyanat, 2-Fluor-4-iodphenylisocyanat, 3-Bromo-2,4,6-trimethylphenylisocyanat, 3-Chlor-2-fluorphenylisocyanat, 3-Chlor-2-methylphenylisocyanat, 4-(Trifluormethyl)benzylisocyanat, 4-Brom-2,6-difluorphenylisocyanat, 4-Brom-2,6-dimethylphenylisocyanat, 4-Brom-2-(trifluormethyl)phenylisocyanat, 4-Brom-2-chlor-6-methylphenylisocyanat, 4-Brom-2-chlor-6-methylphenylisocyanat, 4-Brom-2-ethylphenylisocyanat, 4-Chlor-2-phenoxyphenylisocyanat, 4-Ethoxy-2-nitrophenylisocyanat, 4-Fluor-2-nitrophenylisocyanat, 5-Chlor-2-methylphenylisocyanat, 5-Chlor-2-phenoxyphenylisocyanat, 5-Methyl-2-nitrophenylisocyanat, 5-Phenyl-2-thienylisocyanat, 6-Fluor-4H-1,3-benzodioxin-8-ylisocyanat, 9H-Fluoren-9-ylisocyanat, Benzylisocyanat, Ethylisocyanat, Trichloracetylisocyanat, 1-Phenylethylisocyanat, Ethylisocyanatformat, Isocyanatphosphonicdichlorid, 2-Isocyanatthylmethacrylat, 3-Isocyanat-4-methoxybiphenyl, 2,4,6-Trichlorphenylisocyanat, Triphenylsilylisocyanat, 2,6-Dibrom-4-ethylphenylisocyanat, 2-Chlor-4-nitrophenylisocyanat, 2-tert-Butyl-6-methylphenylisocyanat, 4,4'-Methylenebis(2-chlorphenylisocyanat), 4,5-Dimethyl-2-nitrophenylisocyanat, 4-Chlor-2-(trifluormethyl)phenylisocyanat, 4-Chloro-2-nitrophenylisocyanat, 1-Isocyanat-2,3-dimethoxybenzol, 3-Isocyanatopentan, Isocyanatocyclobutan, Isocyanato(methoxy)methan, Ethyl(4-isocyanatophenyl)acetat, Ethyl4-(isocyanatomethyl)cyclohexancarboxylat, 1,1-Dimethoxy-2-isocyanatoethan, 1-Chlor-3-fluor-2-isocyanatobenzen, 2-Chlor-3-fluorphenylisocyanat, 2-Isocyanato-3-methyl-butyricsäuremethylester, 2-Isocyanato-5-methylbenzonitril, 5-Chlor-2-isocyanatobenzonitril, 5-Ethyl-2-isocyanatobenzonitril, 6-Isocyanato-hexansäuremethylester, Dimethyl-2-isocyanatoterephthalat, Ethyl-2-isocyanat-4-methylvalerat, Methyl-2-isocyanat-4-(methylsulfanyl)butanoat, Methyl-2-isocyanat-4-methylpentanoat, Ethyl-isocyanatoacetat, Phenyl-isocyanatoformat, Methyl-4-isocyanatobenzoat, Methyl-3-isocyanatobenzoat, Methyl-isocyanatoformat, Dimethyl 5-isocyanatoisophthalat und beliebigen Gemischen solcher Monoisocyanate.

Ebenfalls geeignet sind Thioisocyanate. Bevorzugte Thioisocyanate sind ausgewählt aus der Gruppe bestehend aus 4-Fluorbenzylisothiocyanat, Dibutylzinndiisothiocyanat, 2,6-Difluorphenylisothiocyanat, 3-Cyanophenylisothiocyanat, 3-Nitrophenylisothiocyanat und Phenylisocyanat.

Besonders bevorzugt ist ein Monoisocyanat ausgewählt aus der Gruppe bestehend aus Cyclohexylisocyanat, Phenylisocyanat, Octadecylisocyanat und Hexylisocyanat.

Ebenfalls geeignet sind Mono- oder Polyisocyanate, die durch die Modifizierung monomerer Isocyanate wie weiter oben in dieser Anmeldung beschrieben erhalten werden.

### Polymerisierbare Zusammensetzung

In einer weiteren Ausführungsform betrifft die vorliegende Erfindung eine polymerisierbare Zusammensetzung enthaltend
a) wenigstens ein Polyisocyanat mit Isocyanatgruppen ausgewählt aus der Gruppe bestehend aus aliphatisch, cycloaliphatisch, araliphatisch und aromatisch gebundenen Isocyanatgruppen; und
b) wenigstens eine Verbindung gemäß Formel (I); 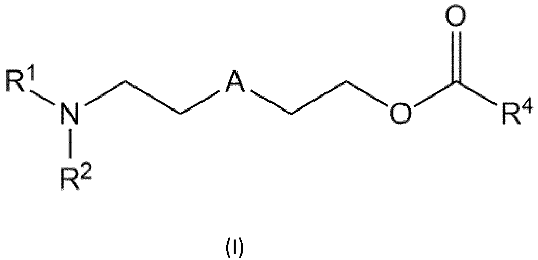
   Wobei R¹ und R² unabhängig voneinander ausgewählt sind aus der Gruppe bestehend aus Wasserstoff, Methyl, Ethyl, Propyl, Isopropyl, Butyl, Isobutyl, verzweigtem C5-Alkyl, unverzweigtem C5-Alkyl, verzweigtem C6-Alkyl, unverzweigtem C6-Alkyl, verzweigtem C7-Alkyl und unverzweigtem C7-Alkyl;
   A ausgewählt ist aus der Gruppe bestehend aus O, S und NR³, wobei R³ ausgewählt ist aus der Gruppe bestehend aus Wasserstoff, Methyl, Ethyl, Propyl, Isopropyl, Butyl und Isobutyl; und
   R⁴ ein verzweigter oder unverzweigter Alkylrest bis zu 7 Kohlenstoffatomen oder Wasserstoff ist; und
wobei das Verhältnis von Isocyanatgruppen zu mit Isocyanatgruppen reaktiven Gruppen in der polymerisierbaren Zusammensetzung wenigstens 2:1 beträgt.

Alle weiter oben in dieser Anmeldung für die erfindungsgemäße Verbindung gegebenen Definitionen gelten auch für diese Ausführungsform.

Die Gesamtheit aller in der polymerisierbaren Zusammensetzung enthaltenen monomeren und oligomeren Polyisocyanate wird in dieser Anmeldung auch als "Polyisocyanatkomponente" der polymerisierbaren Zusammensetzung bezeichnet.

Eine "polymerisierbare Zusammensetzung" ist eine Zusammensetzung, welche wenigstens die oben definierten Komponenten enthält und durch Vernetzung der in der Zusammensetzung enthaltenen freien Isocyanatgruppen zu einem Polymer ausgehärtet werden kann. Hierbei wirkt die Verbindung nach Formel (I) als Katalysator, welche die Vernetzung der Isocyanatgruppen bewirkt.

Diese Vernetzung von wenigstens zwei Isocyanatgruppen erfolgt vorzugsweise unter Ausbildung von Isocyanurat- und/oder Uretdiongruppen. Es entstehen überwiegend Isocyanuratgruppen und Uretdiongruppen stellen nur ein Nebenprodukt dar. Soweit Isocyanuratgruppen gebildet werden, werden vorzugsweise pro gebildeter Isocyanuratgruppe drei Isocyanatgruppen vernetzt.

Das Mengenverhältnis zwischen der Verbindung gemäß Formel (I) auf der einen Seite zu dem wenigstens einen Polyisocyanat mit Isocyanatgruppen ausgewählt aus der Gruppe bestehend aus aliphatisch, cycloaliphatisch, araliphatisch und aromatisch gebundenen Isocyanatgruppen auf der anderen Seite ist so gewählt, dass bei Temperaturen zwischen 80 °C und 250 °C wenigstens 80 % der vorliegenden freien Isocyanatgruppen innerhalb von höchstens einer Stunde vernetzt werden können. Diese Bedingung wird vorzugsweise dann erfüllt, wenn das Gewichtsverhältnis zwischen der Verbindung nach Formel (I) und der Gesamtheit der in der polymerisierbaren Zusammensetzung enthaltenen Polyisocyanate zwischen 1 : 1000 und 1 :20, stärker bevorzugt zwischen 1 : 500 und 1 : 20, noch stärker bevorzugt zwischen 1 : 400 und 1:20 und am stärksten bevorzugt zwischen 1 : 300 und 1 : 20 liegt.

Bevorzugt sind wenigstens 80 %, stärker bevorzugt wenigstens 90 %, noch stärker bevorzugt wenigstens 95 % und besonders bevorzugt wenigstens 98 % der in der polymerisierbaren Zusammensetzung enthaltenen Isocyanatgruppen aliphatisch und/oder cycloaliphatisch gebunden.

Es ist besonders bevorzugt, dass die Polyisocyanatkomponente der polymerisierbaren Zusammensetzung zu wenigstens 80 Gew.-% aus wenigstens einem Polyisocyanat ausgewählt aus der Gruppe bestehend aus monomerem HDI, oligomerem HDI, monomerem PDI, oligomerem PDI, monomerem IPDI und oligomerem IPDI besteht. Ganz besonders bevorzugt besteht sie zu wenigstens 90 Gew.-% aus wenigstens einem der vorgenannten Polyisocyanate.

Bevorzugt beträgt das molare Verhältnis von Isocyanatgruppen zu mit Isocyanatgruppen reaktiven Gruppen in der polymerisierbaren Zusammensetzung wenigstens 5:1 und stärker bevorzugt wenigstens 10:1. Unter "mit Isocyanat reaktiven Gruppen" werden Hydroxyl-, Thiol und Aminogruppen verstanden.

### Kit

In noch einer weiteren Ausführungsform betrifft die vorliegende Erfindung ein Kit enthaltend
a) wenigstens ein Polyisocyanat mit Isocyanatgruppen ausgewählt aus der Gruppe bestehend aus aliphatisch, cycloaliphatisch, araliphatisch und aromatisch gebundenen Isocyanatgruppen; und
b) wenigstens einer Verbindung gemäß Formel (I).

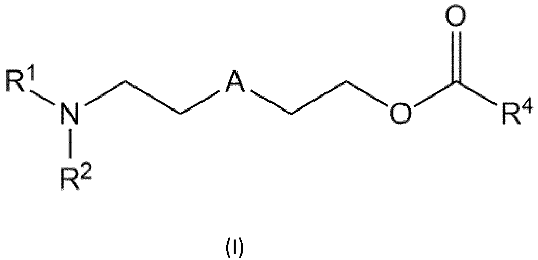

Die Verbindung gemäß Formel (I) wurde weiter oben in dieser Anmeldung definiert.

Das Vorliegen der beiden Komponenten als Kit bedeutet, dass beide Komponenten gemeinsam, aber in getrennten Behältnissen vorliegen. Das Kit enthält in einer bevorzugten Ausführungsform weiterhin eine Bedienungsanleitung, welche die erfindungsgemäße Verwendung beschreibt. Vorzugsweise enthält das Kit die Komponente b) in einer Menge, welche geeignet ist, wenigstens 80 % der Isocyanatgruppen, die in der im Kit enthaltenen Komponente a) vorliegen, bei einer Temperatur zwischen 80 °C bis 250 °C in höchstens 10 Minuten zu vernetzen.

Das Polyisocyant, das im erfindungsgemäßen Kit enthalten ist, entspricht der "Polyisocyanatkomponente" der oben definierten polymerisierbaren Zusammensetzung. Alle dort gegebenen Definitionen gelten deswegen auch für das im Kit enthaltene Polyisocyanat.

### Verwendung

Die vorliegende Erfindung betrifft auch die Verwendung wenigstens einer Verbindung gemäß Formel (I) wie oben definiert 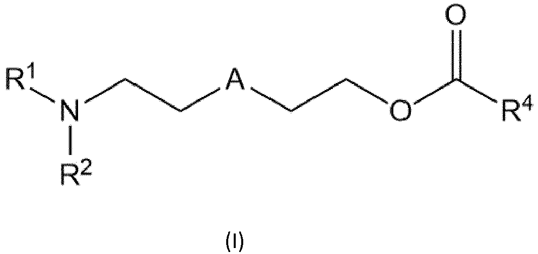 zur Vernetzung von wenigstens zwei Isocyanatgruppen ausgewählt aus der Gruppe bestehend aus aliphatisch, cycloaliphatisch, araliphatisch und aromatisch gebundenen Isocyanatgruppen.

Alle für die polymerisierbare Zusammensetzung oben gegebenen Definitionen gelten auch für diese Ausführungsform, soweit nicht explizit abweichend definiert.

Bevorzugt erfolgt die Vernetzung der wenigstens zwei Isocyanatgruppen ausgewählt aus der Gruppe bestehend aus aliphatisch, cycloaliphatisch, araliphatisch und aromatisch gebundenen Isocyanatgruppen unter Ausbildung einer Isocyanuratgruppe.

In einer besonders bevorzugten Ausführungsform wird die Verbindung gemäß Formel (I) als thermolatenter Katalysator verwendet.

Besonders bevorzugt ist die Verwendung zur Vernetzung von wenigstens zwei aliphatisch und/oder cycloaliphatisch gebundenen Isocyanatgruppen. Ganz besonders bevorzugt werden in wenigstens einer Verbindung ausgewählt aus der Gruppe bestehend aus HDI, PDI, IPDI, oligomerem HDI, oligomerem PDI und oligomem IPDI enthaltene Isocyanatgruppen miteinander vernetzt.

Die erfindungsgemäße Verwendung führt bevorzugt zu einem hochvernetzten Polymer. Im Sinne der Erfindung werden unter chemisch hochvernetzten Polymeren solche verstanden, die eine mittlere chemische Netzbogenlänge Mcvon höchstens 1000g/mol, bevorzugt höchstens 500g/mol , besonders bevorzugt höchstens 400 g/mol und ganz besonders bevorzugt höchstens 300 g/mol haben. Als mittlere Netzbogenlänge ist dabei die zahlenmittlere Molmasse zwischen den Netzknoten in einem Polymernetzwerk definiert.

Die mittlere Netzbogenlänge, sowie auch die Vernetzungsdichte kann dabei über Quellungsmessungen nach der Methode von HERMANS-FLORY-WALL in geeigneten Lösungsmitteln oder über Messung des Elastizitätsmoduls in der Schmelze im linear-elastischen Bereich bei niedrigen Frequenzen berechnet werden, siehe auch John d. Ferry: Viscoelastic properties of polymers 3rd Edition, 1980.

Bevorzugt wird die Netzbogenlänge über eine rheologische Messung ermittelt.

Im Sinne der Erfindung werden unter hochvernetzten Materialien auch solche verstanden, die einen Speicherschubmodul in der Schmelze gemessen im linearen Bereich von wenigstens 3 ×10⁶ Pa bevorzugt wenigstens 5 ×10⁶ Pa und ganz besonders bevorzugt wenigstens 8 × 10⁶ Pa haben.

Die Verwendung als thermolatenter Katalysator ist dadurch gekennzeichnet, dass der Katalysator mit dem zu vernetzenden Isocyanat vermischt wird und das dadurch entstandene Reaktionsgemisch zunächst bei einer Temperatur gelagert wird, bei der der Katalysator keine wesentliche katalytische Aktivität zeigt. Anschließend wird die Temperatur auf einen Wert erhöht, bei dem der Katalysator aktiv ist und die Vernetzungsreaktion so gestartet wird. Die Lagerung erfolgt bevorzugt bei Temperaturen von höchstens 40 °C, stärker bevorzugt höchstens 30 °C. Die Lagerdauer ist so bemessen, dass die Viskosität des Reaktionsgemisches in dieser Zeit um höchstens 200 % ansteigt. Bei Lagertemperaturen von 30 °C ist dies vorzugsweise ein Zeitraum von 30 Minuten bis 5 Tagen, stärker bevorzugt von 30 Minuten bis 24 Stunden. Insbesondere steigt die Viskosität bei einer Lagertemperatur von höchstens 30 °C innerhalb von 4 Stunden um höchstens 20 % an. Zur Aktivierung des Katalysators wird die Temperatur auf 50 °C bis 250 °C erhöht, bevorzugt auf 80 °C bis 250 °C und stärker bevorzugt auf 120 °C bis 250 °C.

Weiterhin betrifft die vorliegende Erfindung die Verwendung einer polymerisierbaren Zusammensetzung wie weiter oben definiert oder eines Kits wie weiter oben definiert zur Herstellung eines Polymers.

Besagtes Polymer ist vorzugsweise das Matrixmaterial eines Verbundwerkstoffs. Besonders bevorzugt ist es das Matrixmaterial eines hochgefüllten Verbundwerkstoffs.

Der Begriff "Verbundwerkstoff" ist dem Fachmann gut bekannt, grundsätzlich handelt es sich um Werkstoffe, bei denen ein Füllstoff in eine Matrix eingebettet ist. Erfindungsgemäß ist diese Matrix ein Polymer, das durch die Vernetzung der in der Polyisocyanatkomponente a) enthaltenen Isocyanatgruppen entsteht.

Der Füllstoff kann jeder dem Fachmann bekannte, geeignete organische oder anorganische Füllstoff sein. Er kann jede beliebige Geometrie aufweisen. Vorzugsweise handelt es sich aber um einen faserförmigen organischen oder anorganischen Füllstoff.

Das Aspektverhältnis eines faserförmigen Füllstoffs ist größer als 1000, bevorzugt größer als 5000, stärker bevorzugt größer als 10.000 und am stärksten bevorzugt größer als 50.000. Das Aspektverhältnis ist definiert als die Länge der Faser geteilt durch den Durchmesser.

Die faserförmigen Füllstoffe weisen bei Einhaltung des oben definierten Aspektverhältnisses bevorzugt eine Mindestlänge von 2 mm, stärker bevorzugt 5 mm, noch stärker bevorzugt 20 mm, noch stärker bevorzugt 100 mm, am stärksten bevorzugt 1000 mm, besonders bevorzugt 50 m und ganz besonders bevorzugt 100 m auf.

Bevorzugte anorganische Fasern sind Glasfasern, Basaltfasern, Borfasern, Keramikfasern, Whisker, Kieselsäurefasern sowie metallische Verstärkungsfasern. Bevorzugte organische Fasern sind Aramidfasern, Kohlenstofffasern, Kohlenstoffnanoröhrchen, Polyester-Fasern, Nylon-Fasern sowie Plexiglas-Fasern. Bevorzugte Naturfasern sind Flachsfasern, Hanffasern, Holzfasern, Cellulosefasern sowie Sisalfasern.

Das Verhältnis der Mengenanteile von Füllstoff und Polymermatrix im Verbundwerkstoff wird als Füllungsgrad bezeichnet. Hochgefüllte Systeme zeichnen sich durch einen Gewichtsanteil des Füllstoffs zwischen 50 Gew.-% und 90 Gew.-%, bevorzugt zwischen 60 Gew.-% und 85 Gew.-% und noch stärker bevorzugt zwischen 70 und 85 Gew.-% aus, immer bezogen auf die Gesamtmasse von Polyisocyanatkomponente a) und Füllstoff.

### Verfahren

In noch einer weiteren Ausführungsform betrifft die vorliegende Erfindung ein Verfahren zur Herstellung eines Polymers enthaltend die Schritte
a) Vermischen wenigstens eines Polyisocyanats mit Isocyanatgruppen ausgewählt aus der Gruppe bestehend aus aliphatisch, cycloaliphatisch, aromatisch und araliphatisch gebundenen Isocyanatgruppen mit wenigstens einer Verbindung gemäß Formel (I); 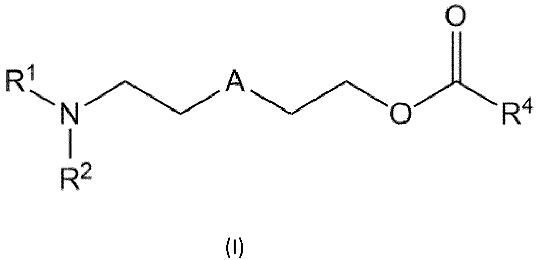
b) Aushärten der in Verfahrensschritt a) erhaltenen polymerisierbaren Zusammensetzung durch Erhöhung der Temperatur auf wenigstens 50 °C,
wobei zu Beginn des Verfahrensschritts b) das Verhältnis von Isocyanatgruppen zu mit Isocyanat reaktiven Gruppen in der polymerisierbaren Zusammensetzung wenigstens 2:1 beträgt.

Alle Definitionen der erfindungsgemäß einsetzbaren Polyisocyanate und der Verbindung gemäß Formel (I), die weiter oben in dieser Anmeldung gegeben wurden, gelten auch für diese Ausführungsform.

Das Vermischen der Komponenten in Verfahrensschritt a) kann mit allen dem Fachmann bekannten geeigneten Verfahren erfolgen. Der Verfahrensschritt a) wird vorzugsweise bei Temperaturen von höchstens 40 °C durchgeführt. Am Ende des Verfahrensschritts a) liegt ein Reaktionsgemisch vor, das der am Anfang dieser Patentanmeldung offenbarten polymerisierbaren Zusammensetzung entspricht. Insofern offenbart der Verfahrensschritt a) für sich alleine betrachtet ein Verfahren zur Bereitstellung der erfindungsgemäßen polymerisierbaren Zusammensetzung.

Geeignete Mengenverhältnisse der Komponenten sind weiter oben in Zusammenhang mit der polymerisierbaren Zusammensetzung beschrieben. Insbesondere beträgt das Verhältnis von Isocyanatgruppen zu mit Isocyanatgruppen reaktiven Gruppen in der polymerisierbaren Zusammensetzung wenigstens 5:1 und stärker bevorzugt wenigstens 10:1. Unter "mit Isocyanat reaktiven Gruppen" werden Hydroxyl-, Thiol und Aminogruppen verstanden.

Da die Verbindung gemäß Formel (I) ein thermolatenter Katalysator ist, wird der Verfahrensschritt b) vorzugsweise durch eine Temperaturerhöhung der polymerisierbaren Zusammensetzung eingeleitet. Die erhöhte Temperatur wird vorzugsweise auch über die Gesamtdauer des Verfahrensschrittes b) gehalten. Es ist bevorzugt, dass während des gesamten Verfahrensschrittes b) eine Temperatur von wenigstens 50 °C gehalten wird. Stärker bevorzugt liegt die Temperatur während des Verfahrensschrittes b) zwischen 50 °C und 250 °C, noch stärker bevorzugt zwischen 80 °C und 250 °C und am stärksten bevorzugt zwischen 120 °C und 250 °C.

Da die Verbindung gemäß Formel (I) bei Temperaturen bis 30 °C keine wesentliche katalytische Aktivität zeigt, kann die in Verfahrensschritt a) erhaltene polymerisierbare Zusammensetzung vor Beginn des Verfahrensschritts b) für 30 bis 360, bevorzugt 30 bis 249 Minuten und unter Einhaltung dieser Temperaturgrenze gelagert werden, ohne dass ihre Viskosität um mehr als 20 % ansteigt.

Das "Aushärten" der polymerisierbaren Zusammensetzung erfolgt durch die Vernetzung der im Polyisocyanat enthaltenen Isocyanatgruppen. Hierdurch entsteht ein festes Polymernetzwerk. Da die Verbindung gemäß Formel (I) vor allem die Ausbildung von Isocyanuratgruppen katalysiert, werden vorzugsweise zu wenigstens 80 mol-% Isocyanuratgruppen ausgebildet, während die Summe an Uretdion-, Urethan-, Allophanat-, Harnstoff-, Biuret-, Iminooxadiazindion- und Oxadiazintrionstrukturen, die während des Aushärtens entstehen, unter 20 mol-% liegt.

Der Verfahrensschritt b) ist abgeschlossen, wenn aus der flüssigen polymerisierbaren Zusammensetzung ein Feststoff entstanden ist, der ohne äußere Stützen wie z.B. Gießformen seine Form behält. Dies ist vorzugsweise der Fall, wenn wenigstens 75 %, stärker bevorzugt wenigstens 80 %, noch stärker bevorzugt wenigstens 85 % und am stärksten bevorzugt wenigstens 90 % der zu Beginn des Verfahrensschrittes b) in der polymerisierbaren Zusammensetzung vorliegenden Isocyanatgruppen verbraucht sind. Bei Temperaturen zwischen 80 °C und 250 °C ist dieser Zustand vorzugsweise nach maximal 20 Minuten erreicht.

Wenn die Herstellung eines Verbundwerkstoffs beabsichtigt ist, muss die polymerisierbare Zusammensetzung zu Beginn des Verfahrensschritts b) einen Füllstoff enthalten. Dieser kann auf unterschiedlichen Wegen in die polymerisierbare Zusammensetzung eingebracht werden. Die polymerisierbare Zusammensetzung, wie sie am Ende des Verfahrensschrittes a) vorliegt, kann mit dem Füllstoff vermischt werden. Es ist aber auch möglich ein Polyisocyanat mit Isocyanatgruppen ausgewählt aus der Gruppe bestehend aus aliphatisch, cycloaliphatisch, araliphatisch und aromatisch gebundenen Isocyanatgruppen zunächst mit dem Füllstoff zu vermischen, bevor es in Verfahrensschritt a) eingesetzt wird. Geeignete Füllstoffe sind weiter oben in dieser Anmeldung beschrieben. In Verfahrensschritt b) entsteht so ein Verbundwerkstoff, bei dem das ausgehärtete Reaktionsgemisch eine Polymermatrix bildet, in die der Füllstoff eingebettet ist.

Gemäß einer bevorzugten Ausführungsform der Erfindung ist der Füllstoff ein faserförmiger Füllstoff, ausgewählt aus der Gruppe bestehend aus Glasfasern, Basaltfasern, Kohlenstofffasern, Cellulosefasern und Mischungen daraus. Der Begriff "Cellulosefaser" bezeichnet vorzugsweise Fasern, die Cellulose enthalten. Es ist dabei nicht ausgeschlossen, dass sie weitere Verbindungen wie z.B. Pektine enthalten. Da erfindungsgemäß bevorzugte Cellulosefasern pflanzlicher Herkunft sind, hängt die genaue chemische Struktur der Fasern von der betreffenden Pflanze ab. Bevorzugte Cellulosefasern sind Fasern aus Jute, Flachs, Hanf und Baumwolle. Die Fasern können einzeln vorliegen, sie können aber auch in jeder dem Fachmann bekannten Form zu Matten oder Fliesen gewebt oder gewirkt vorliegen. Vorzugsweise liegen weniger als 50 Gew.-%, stärker bevorzugt weniger als 35 Gew.-%, noch stärker bevorzugt weniger als 20 Gew.-% und am stärksten bevorzugt weniger als 10 Gew.-% der verwendeten Fasern in Form von Matten oder Fliesen vor.

In einer besonders bevorzugten Ausführungsform der vorliegenden Erfindung ist das erfindungsgemäße Verfahren zur Herstellung eines Verbundwerkstoffs ein Strangziehverfahren, auch unter dem Begriff Pultrusionsverfahren bekannt.

Das Strangziehen ist ein kontinuierliches Herstellungsverfahren zur Fertigung von faserverstärkten Kunststoffprofilen. Der prinzipielle Aufbau einer Strangziehanlage besteht aus dem Faserregal, Vorrichtungen für die Faserführung, Imprägniereinrichtung, Härtungswerkzeug, reziprok- arbeitenden Ziehvorrichtungen und einer Ablängeinheit.

Die Spulen der Rovings werden im Faserregal gelagert. Von dort werden die Faserrovings über Faserführungen zur Imprägniereinrichtung geführt, wo die Fasern mit dem Reaktionsgemisch aus Verfahrensschritt a) benetzt werden. Die Fasern werden in der Regel über die Faserführungen oder auch die Imprägniervorrichtung bereits an die später gewünschte Profilform ausgerichtet bzw. vorsortiert. Besonders geeignet sind Fasern, die unter Einhaltung des oben definierten Aspektverhältnisses eine Mindestlänge von wenigstens 50 m aufweisen.

Zudem können wenn erforderlich Matten, Gewebe, Gelege oder Vliese in den Prozess integriert werden, um die mechanischen Eigenschaften für die gewünschte Anwendung zu optimieren. Die Imprägnierung der Fasern mit dem Reaktionsgemisch kann durch alle dem Fachmann im Kontext des Strangziehverfahrens bekannten Methoden erfolgen.

Anschließend durchlaufen die harzgetränkten Fasern das formgebende Härtungswerkzeug, wo die Vernetzung der reaktiven Gruppen des Reaktionsgemisches zum Polymer (Matrix) durch erhöhte Temperatur erfolgt. Dies ist der Verfahrensschritt b) des Verfahrens. Im Anschluss folgt häufig eine Kühlstrecke, z.B. Luftkühlung, bevor das nun fertige Halbzeug durch die alternierend arbeitenden Ziehvorrichtungen (puller) geführt wird. Diese sorgen für einen kontinuierlichen Transport des Materials im gesamten Strangziehprozess. Als letzter Prozessschritt wird das Material auf die gewünschte Länge zugeschnitten. Häufig kommt hier eine ,fliegende Säge' zum Einsatz, d.h. dass die Säge mit der gleichen Geschwindigkeit mit dem Material mitfährt und dabei den Schnitt durchführt. Auf diese Art erhält man eine gerade Schnittkante und verhindert ein Aufstauen des Profils oder anhalten des Prozesses während des Sägeschritts.

Weiterhin betrifft die vorliegende Patentanmeldung ein Polymer, das durch das oben beschriebene Verfahren erhältlich ist.

Gegenüber den im Stand der Technik, insbesondere in WO 2019/197638 und WO 2019/197639 beschriebenen Katalysatoren ergeben sich bei Einsatz der erfindungsgemäßen Katalysatoren mehrere Vorteile. Bei Zugabe der erfindungsgemäßen Katalysatoren zu einer Polyisocyanatzusammensetzung ist die Viskosität des erhaltenen Reaktionsgemisches sowohl unmittelbar nach der Zugabe als auch mehrere Stunden danach geringer als bei Verwendung der aus dem Stand der Technik bekannten Katalysatoren.

Die nachfolgenden Ausführungsbeispiele dienen nur dazu, die Erfindung zu illustrieren. Sie sollen den Schutzbereich der Patentansprüche in keiner Weise beschränken.

### Ausführungsbeispiele

### Allgemeine Hinweise:

Alle Prozentangaben beziehen sich, soweit nicht anders angegeben, auf Gewichtsprozente (%).

Die Umgebungstemperatur von 23°C zum Zeitpunkt der Durchführung der Versuche wird als RT (Raumtemperatur) bezeichnet.

Die nachfolgend beschriebenen Verfahren zur Bestimmung der relevanten Parameter wurden zur Durchführung/Auswertung der Beispiele eingesetzt und sind auch allgemein die Verfahren zur Bestimmung der erfindungsgemäß relevanten Parameter.

### Ausgangsverbindungen:

Polyisocyanat, Desmocomp Ultra AP 200 (NCO-Funktionalität > 3) mit einem NCO-Gehalt von 23,0 Gew.-% der Fa. Covestro AG. Die Viskosität beträgt ca. 1200 mPa ▪ s bei 23°C (DIN EN ISO 3219/A.3). Katalysator 1: N,N,N'-Trimethylaminoethylethanolamin mit einer OH-Zahl von 384 mg KOH/g wurde von der Huntsman Corporation mit dem Handelsnamen Jeffcat-Z110 erhalten.

Katalysator 2: Katalysatoraddukt wie in WO 2019/197638 als Katalysator KA1 beschrieben Zusatzmittel: Entformungsmittel Desmorapid AP 300, der Fa. Covestro AG, besteht aus organischen Fettsauren und Estern.

### Experimentelles Verfahren zur Synthese von Katalysator 3:

Nach Abkühlen einer Lösung von 10,0 g 2-[2-(Dimethylamino)-ethyl]-methylamino}-ethanol und 9,53 ml Triethylamin in 100 ml Essigsäureethylester wurden 5,35 ml Acetylchlorid langsam zugegeben, wobei die Temperatur zwischen 0°C und 50°C gehalten wurde, wodurch die Ausfällung von Triethylaminhydrochlorid induziert wurde. Man ließ das Reaktionsgemisch 15 Minuten bei 0°C und 24 Stunden bei Raumtemperatur rühren. Das Reaktionsgemisch wurde filtriert und der Niederschlag mit Essigester gewaschen. Das Lösungsmittel wurde im Vakuum eingedampft und durch Vakuumdestillation des Rohproduktes erhielt man das Produkt als farblose Flüssigkeit (10,30 g, 80 % Ausbeute) (>95 % Reinheit).

### Herstellung des Reaktionsgemisches

Sofern nicht anders angegeben, wurde das Reaktionsgemisch durch Mischen des Polyisocyanat (Desmocomp Ultra AP 200) mit einer entsprechenden Menge an Katalysator und Additiv bei 23°C in einem Speedmixer DAC 150.1 FVZ der Firma Hauschild bei 2750 RPM hergestellt.

### Zusammensetzung der Beispiele

### Vergleichsbeispiel 1

Zu 99,5 Gew.-% Polyisocyanat wurden 0,5 Gew.-% Katalysator 1 gegeben und gemäß oben ausgeführter Vorgehensweise zubereitet.

### Vergleichsbeispiel 2

Zu 98,8 Gew.-% Polyisocyanate wurden 1,2 Gew.-% Katalysator 2 gegeben und gemäß oben ausgeführter Vorgehensweise zubereitet.

### Erfindungsgemäßes Beispiel

Zu 97,5 Gew.& Polyisocyanat wurden 2,5 Gew.-% Katalysator 3 gegeben und gemäß oben ausgeführter Vorgehensweise zubereitet.

### Messmethoden:

### Phasenübergang mittels DSC:

Bestimmung der Phasenübergänge mittels DSC: Die Phasenübergänge wurde mittels DSC (Differential Scanning Calorimetry) mit einem Mettler DSC 12E (Mettler Toledo GmbH, Gießen, DE) entsprechend DIN EN 61006 bestimmt. Eine Kalibrierung erfolgte durch die Temperatur des Schmelz-Onsets von Indium und Blei. Es wurden 5 mg Substanz in Normalkapseln eingewogen. Die Messung erfolgte durch zwei Aufheizungen von -20 °C bis +200 °C bei einer Heizrate von 20 K/min mit anschließender Abkühlung mit einer Kühlrate von 320 K/min. Die Kühlung erfolgte durch flüssigen Stickstoff. Als Spülgas wurde Stickstoff verwendet. Die angegebenen Werte basieren jeweils auf der Auswertung der 2. Aufheizkurve. Die Schmelztemperaturen Tm wurden aus den Temperaturen an den Maxima der Wärmeflusskurven erhalten. Die Glasübergangstemperatur Tg wurde aus der Temperatur bei der halben Höhe einer Glasübergangsstufe erhalten.

### 4 Stunden Viskosität:

Die Viskosität einer kleinen Menge des reaktiven Harzes wurde bei 23 °C mit einem Physia MCR 51 der Firma Anton Paar (platte/platte; Scherrrate 1 s-1) über 4 h gemessen. Wie auch in der WO2019121349A1 beschrieben und in Anlehnung an ISO 6721-10.

**Tabelle 1: Viskositätsentwicklung über 4 Stunden der aktivierten Matrixsysteme**

| Zeit in Minuten | Vergleichsbeispiel 1 Viskosität bei 23°C | Vergleichsbeispiel 2 | erfindungsgemäßes Beispiel |
|---|---|---|---|
| 0 | 1220 mPa^{∗}s | 1300 mPa^{∗}s | 897 mPa^{∗}s |
| 30 | 1240 mPa^{∗}s | 1310 mPa^{∗}s | 922 mPa^{∗}s |
| 60 | 1270 mPa^{∗}s | 1320 mPa^{∗}s | 924 mPa^{∗}s |
| 90 | 1280 mPa^{∗}s | 1330 mPa^{∗}s | 928 mPa^{∗}s |
| 120 | 1290 mPa^{∗}s | 1340 mPa^{∗}s | 931 mPa^{∗}s |
| 150 | 1300 mPa^{∗}s | 1340 mPa^{∗}s | 934 mPa^{∗}s |
| 180 | 1310 mPa^{∗}s | 1350 mPa^{∗}s | 942 mPa^{∗}s |
| 210 | 1320 mPa^{∗}s | 1360 mPa^{∗}s | 940 mPa^{∗}s |
| 240 | 1320 mPa^{∗}s | 1360 mPa^{∗}s | 946 mPa^{∗}s |

Die Tabelle 1 zeigt die Viskositätsentwicklung der Vergleichsbeispiele und des erfindungsgemäßen Beispiel im direkten Vergleich über einen Zeitraum von 240 Minuten. Es ist deutlich erkennbar, dass die aktivierte Matrix des erfindungsgemäßen Beispiels einen über den gesamten Messzeitraum stabile und niedrigere Viskosität aufweist, die unterhalb der Ausgangsviskosität des verwendeten Polyisocyanat Desmocomp AP 200 liegt.

**Tabelle 2: Glasübergangstemperaturen der ausgehärteten Matrixsysteme**

| Aktiviertes Matrixsystem | Vergleichsbeispiel 1 | Vergleichsbeispiel 2 | Erfindungsgemäßes Beispiel |
|---|---|---|---|
| Glasübergangstemperatur (Tg) gemäß DSC nach Aushärtung | 99°C | 111°C | 111°C |

In Tabelle 2 sind jeweils die gemessenen Glasübergangstemperaturen (Tg) nach der zweiten Aufheizung der beiden Vergleichsbeispiel 1 und 2 als auch die des erfindungsgemäßen Beispiels zusammengefasst. Alle Tg-Werte wurden von Proben bestimmt, die eine Aushärtung über 10 Minuten bei 160°C erfahren haben.

Die hier aufgeführten Untersuchungen zeigen, dass es durch die Esterfunktionalisierung tertiärer Amine gelungen ist, duroplastische Verbundwerkstoffe im Strangziehverfahren herzustellen, welche eine hohe Glasübergangstemperatur in Kombination mit guter Faseranbindung zeigen.

## Patentansprüche

1. Verbindung gemäß Formel (I) 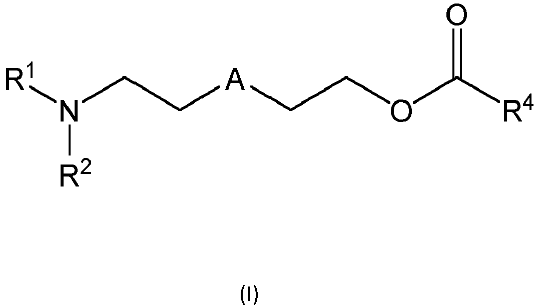
Wobei R¹ und R² unabhängig voneinander ausgewählt sind aus der Gruppe bestehend aus Wasserstoff, Methyl, Ethyl, Propyl, Isopropyl, Butyl, Isobutyl, verzweigtem C5-Alkyl, unverzweigtem C5-Alkyl, verzweigtem C6-Alkyl, unverzweigtem C6-Alkyl, verzweigtem C7-Alkyl und unverzweigtem C7-Alkyl;
A ausgewählt ist aus der Gruppe bestehend aus O, S und NR³, wobei R³ ausgewählt ist aus der Gruppe bestehend aus Wasserstoff, Methyl, Ethyl, Propyl, Isopropyl, Butyl und Isobutyl; und
R⁴ ein verzweigter oder unverzweigter Alkylrest bis zu 7 Kohlenstoffatomen oder Wasserstoff ist.

2. Die Verbindung gemäß Anspruch 1, wobei in Formel (I) A NR³ ist, und R³ ausgewählt ist aus der Gruppe bestehend aus Wasserstoff, Methyl, Ethyl, Propyl, Isopropyl, Butyl und Isobutyl.

3. Die Verbindung gemäß Anspruch 1, wobei in Formel (I) A Sauerstoff ist.

4. Die Verbindung gemäß eines der Ansprüche 1 bis 3, wobei R⁴ ausgewählt ist aus der Gruppe bestehend aus Wasserstoff, Methyl und Ethyl.

5. Verwendung einer Verbindung gemäß eines der Ansprüche 1 bis 4 als Katalysator zur Vernetzung von wenigstens zwei Isocyanatgruppen ausgewählt aus der Gruppe bestehend aus aliphatisch, cycloaliphatisch, araliphatisch und aromatisch gebundenen Isocyanatgruppen.

6. Die Verwendung gemäß Anspruch 5, wobei die Verwendung zu einem hochvernetzten Polymer führt.

7. Polymerisierbare Zusammensetzung enthaltend
a) wenigstens ein Polyisocyanat mit Isocyanatgruppen ausgewählt aus der Gruppe bestehend aus, aliphatisch, cycloaliphatisch, araliphatisch und aromatisch gebundenen Isocyanatgruppen; und
b) wenigstens eine Verbindung gemäß eines der Ansprüche 1 bis 5;
wobei das molare Verhältnis von Isocyanatgruppen zu mit Isocyanatgruppen reaktiven Gruppen in der polymerisierbaren Zusammensetzung wenigstens 2 : 1 beträgt.

8. Die polymerisierbare Zusammensetzung gemäß Anspruch 7, wobei die Polyisocyanatkomponente der polymerisierbaren Zusammensetzung zu wenigstens 80 Gew.-% aus wenigstens einem Polyisocyanat ausgewählt aus der Gruppe bestehend aus monomerem HDI, oligomerem HDI, monomerem PDI, oligomerem PDI, monomerem H12MDI, oligomerem H12MDI,monomerem IPDI und oligomerem IPDI besteht.

9. Kit enthaltend
a) wenigstens ein Polyisocyanat mit Isocyanatgruppen ausgewählt aus der Gruppe bestehend aus aliphatisch, cycloaliphatisch, araliphatisch und aromatisch gebundenen Isocyanatgruppen und
b) wenigstens eine Verbindung gemäß eines der Ansprüche 1 bis 5.

10. Die Verwendung gemäß der polymerisierbaren Zusammensetzung gemäß Anspruch 7 oder 8 oder des Kits gemäß Anspruch 9 zur Herstellung eines Polymers.

11. Verfahren zur Herstellung eines Polymers enthaltend die Schritte
a) Vermischen wenigstens eines Polyisocyanats mit Isocyanatgruppen ausgewählt aus der Gruppe bestehend aus aliphatisch, cycloaliphatisch, araliphatisch und aromatisch gebundenen Isocyanatgruppen mit einer Verbindung gemäß eines der Ansprüche 1 bis 5; und
b) Aushärten der in Verfahrensschritt a) erhaltenen polymerisierbaren Zusammensetzung durch Erhöhung der Temperatur auf wenigstens 50 °C,
wobei zu Beginn des Verfahrensschritts b) das Verhältnis von Isocyanatgruppen zu mit Isocyanat reaktiven Gruppen in der polymerisierbaren Zusammensetzung wenigstens 2:1 beträgt.

12. Das Verfahren nach Anspruch 11, wobei zwischen dem Ende des Verfahrensschritts a) und dem Beginn des Verfahrensschritts b) ein Zeitraum zwischen 30 Minuten und 360 Minuten liegt.

13. Das Verfahren nach Anspruch 11 oder 12, **dadurch gekennzeichnet, dass** das in Verfahrensschritt a) erhaltene Reaktionsgemisch vor Durchführung des Verfahrensschritts b) mit einem organischen oder anorganischen Füllstoff vermischt wird.

14. Das Verfahren nach Anspruch 13, **dadurch gekennzeichnet, dass** der organische oder anorganische Füllstoff aus Fasern mit einer Mindestlänge von 2 mm besteht und die Aushärtung in Verfahrensschritt b) in einem beheizten Werkzeug stattfindet, das dem mit dem Reaktionsgemisch benetzten Faserbündel ein Profil verleiht und dieses Profil durch die Aushärtung des Reaktionsgemisches stabilisiert.

15. Ein Polymer, erhältlich durch das Verfahren gemäß Anspruch 11 oder 12 oder ein Verbundwerkstoff, erhältlich nach dem Verfahren gemäß Anspruch 13 oder 14.
